(19) Europäisches Patentamt
European Patent Office
Office européen des brevets

(11) EP 4 296 259 A1

(12) **EUROPEAN PATENT APPLICATION**
published in accordance with Art. 153(4) EPC

(43) Date of publication:
**27.12.2023 Bulletin 2023/52**

(21) Application number: **22756396.2**

(22) Date of filing: **25.01.2022**

(51) International Patent Classification (IPC):
***C07C 235/76*** *(2006.01)* ***C07C 231/12*** *(2006.01)*
***A61K 8/68*** *(2006.01)* ***A23L 33/10*** *(2016.01)*
***A61K 31/164*** *(2006.01)* ***A61P 17/00*** *(2006.01)*
***A61Q 19/00*** *(2006.01)* ***A61K 39/00*** *(2006.01)*

(52) Cooperative Patent Classification (CPC):
**A23L 33/10; A61K 8/68; A61K 31/164; A61K 31/231; A61K 39/00; A61P 17/00; A61Q 19/00; C07C 231/12; C07C 235/76**

(86) International application number:
**PCT/KR2022/001289**

(87) International publication number:
**WO 2022/177192 (25.08.2022 Gazette 2022/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.02.2021 KR 20210021447**

(71) Applicant: **LCS Biotech Co., Ltd.**
**Suwon-si, Gyeonggi-do 16614 (KR)**

(72) Inventors:
- **PARK, Chang Seo**
  **Anyang-si, Gyeonggi-do 14076 (KR)**
- **LEE, Eun Ok**
  **Seoul 06517 (KR)**
- **KIM, Jin Wook**
  **Yongin-si, Gyeonggi-do 16832 (KR)**

(74) Representative: **Schön, Christoph**
**Dr. Schön, Neymeyr & Partner mbB**
**Bavariaring 26**
**80336 München (DE)**

Remarks:
The complete document including Reference Table(s) and the Sequence Listing(s) can be downloaded from the EPO website

(54) **NOVEL CERAMIDE, PREPARATION METHOD THEREFOR, AND USE THEREOF**

(57) Disclosed herein are disclosed a novel ceramide and, specifically, 1-O-acylceramide having a structure in which a phytosphingosine base has fatty acid chains attached thereto in opposite directions, an isomer thereof, a salt thereof, a solvate thereof, or a hydrate thereof, a use thereof for enhancing a skin barrier function, and a preparation method therefor. 1-O-acylceramide with a purity of 70% or higher can be prepared using the method and is capable of reinforcing a skin barrier function, recovery or amelioration from a weakened skin barrier function, and treating or preventing diseases caused by a decrease in skin barrier function, thereby finding applications in various cosmetics and therapeutic formulations for skin disease.

EP 4 296 259 A1

[FIG. 7]

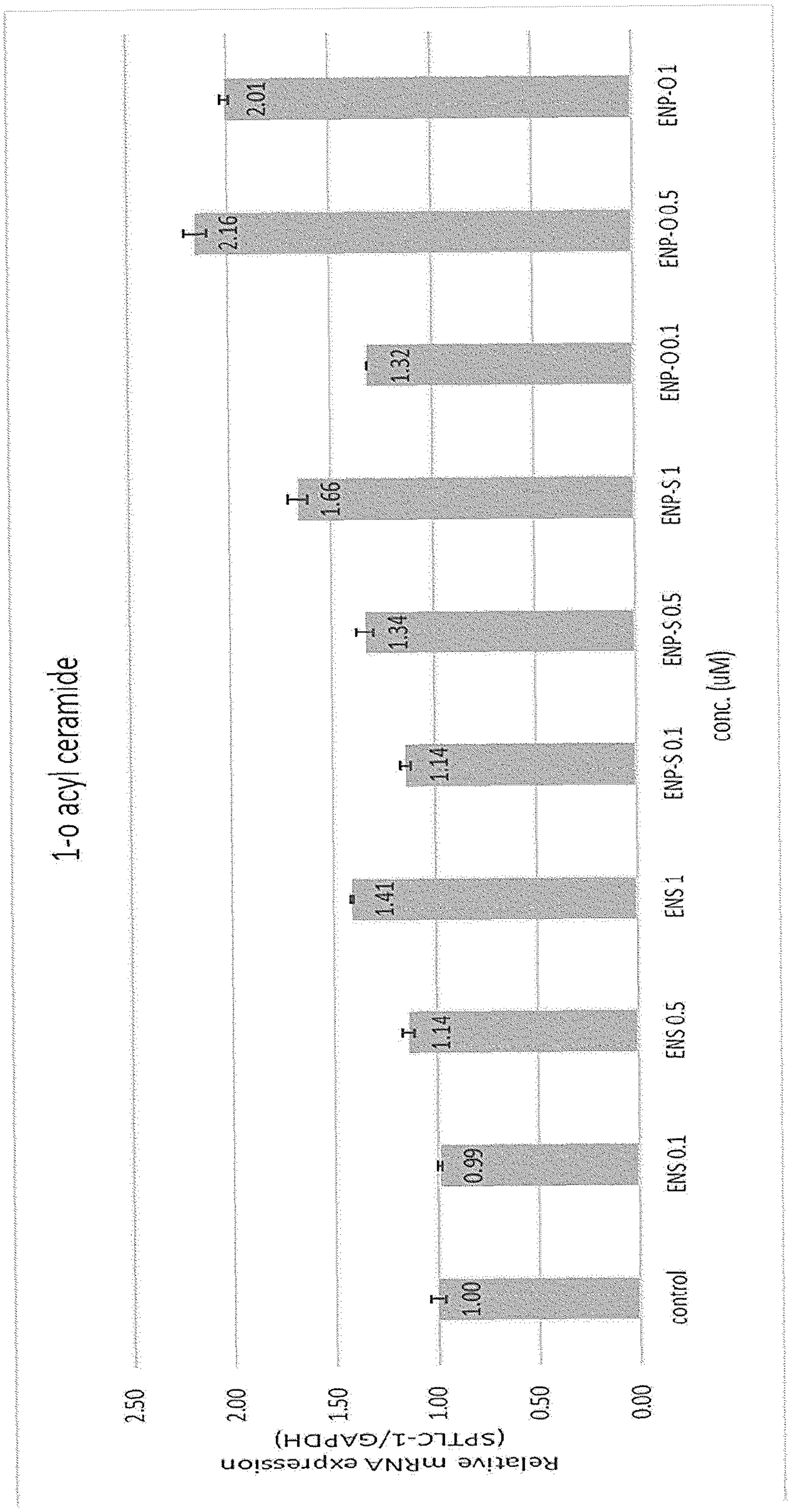
1-o acyl ceramide
Relative mRNA expression
(SPTLC-1/GAPDH)
0.00
0.50
1.00
1.50
2.00
2.50
control
ENS 0.1
ENS 0.5
ENS 1
ENP-S 0.1
ENP-S 0.5
ENP-S 1
ENP-O 0.1
ENP-O 0.5
ENP-O 1
conc. (uM)
1.00
0.99
1.14
1.41
1.14
1.34
1.66
1.32
2.16
2.01

## Description

### [Technical Field]

The technology disclosed herein relates to a novel ceramide, preparation method therefore, and use thereof.

[CROSS REFERENCE TO RELATED APPLICATION]

The present application claims priority to Korean Patent Application No. 10-2021-0021447 filed on February 17, 2021, the entire contents of which are incorporated herein by this reference.

[Government-Sponsored Research and Development]

This study relates to the leading technology development (R&D) project for dermatological application materials (Task serial number: 1465031382), which was conducted by LCS Biotech Co., Ltd. under the support of the Ministry of Health and Welfare and the management of the Korea Health Industry Development Institute. Detailed task number is HP20C0018010020, and the title of the research task is the development of new ceramide production technology and physiological activity.

### [Background Art]

Ceramides, which account for 40 to 50 % of human skin barrier lipids, play a key role in moisturizing and protecting the skin. Ceramides are representative substances of sphingolipids, play various structural roles in the cells of the human body and have important physiological activities necessary for cells to perform normal functions. All sphingolipids refer to lipids with a sphingoid backbone made by polymerization of fatty acids (e.g., palmitic acid) and amino acids (e.g., serine). It has been reported that four types of sphingoid backbones exist: dihydrosphingosine, sphingosine, phytosphingosine, and 6-hydroxysphingosine, depending on the hydroxy group and amino group attached to the head group of the backbone and the presence or absence of double bonds. Ceramides are those in which fatty acids are bonded to amino groups of four types of sphingoid backbones, and are classified into various classes according to the types of fatty acids bonded. Ceramides have a variety of molecular structures, and new classes of ceramides are continually being discovered. Therefore, it is known that more than hundreds of classes of ceramides exist in actual skin (International Journal of Cosmetic Science, 2017, 39, 366-372). Ceramides of various structures and lengths protect the human body by tightly filling the skin barrier and holding each other.

Ceramides have been reported as substances most closely related to various skin diseases and skin troubles, and changes in ceramides have been observed in most skin troubles. Recently, due to the development of analysis technology, it is possible to track a change in each ceramide. That is, it has been reported early that a decrease in the total content of ceramide in various skin diseases such as atopy is a cause of barrier disruption. Recently, it has been reported that ceramides, NP, NH, EOS, EOP, ω-hydroxyceramide, etc. among ceramides decrease significantly, while ceramides, NS, AP, etc. increase, so the importance of individual ceramides is increasingly emphasized.

### [Disclosure]

### [Technical Problem]

In one aspect, the present disclosure is to provide a novel ceramide.

In one aspect, the present disclosure is to provide a novel 1-O-acylceramide.

In one aspect, the present disclosure is to provide a composition for enhancing skin barrier function.

In one aspect, the present disclosure is to provide a method for preparing a novel 1-O-acylceramide.

### [Technical Solution]

In one aspect, the present disclosure relates to a 1-O-acylceramide having a structure of Chemical Formula 1 below, an isomer thereof, a salt thereof, a solvate thereof, or a hydrate thereof.

[Chemical Formula 1]

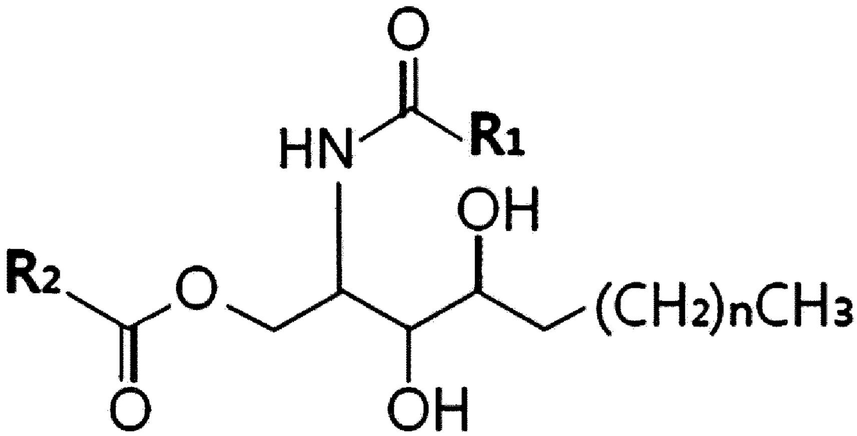

In the above Chemical Formula, $R_1$ and $R_2$ are each independently a C9 to C31 saturated or unsaturated aliphatic chain, and n is an integer of 10 to 20.

In one aspect, the present disclosure relates to a method for preparing the 1-O-acylceramide, the isomer thereof, the salt thereof, the solvate thereof, or the hydrate thereof, comprising binding a fatty acid to a ceramide of a phytosphingosine backbone.

In one aspect, the present disclosure relates to a composition for reinforcing skin barrier function, comprising the 1-O-acylceramide, the isomer thereof, the salt thereof, the solvate thereof, or the hydrate thereof as an active ingredient.

**[Advantageous Effects]**

In one aspect, a novel 1-O-acylceramides can be obtained with the present disclosure.

In one aspect, a high-purity 1-O-acylceramide having a purity of 70 % or more can be obtained with the present disclosure.

In one aspect, it is capable of reinforcing skin barrier function, recovering or ameliorating weakened skin barrier function, or treating or preventing diseases caused by reduction in skin barrier function with the present disclosure.

In one aspect, it is possible to provide excellent ceramide that can be used in various cosmetics and therapeutic formulations for a skin disease with the present disclosure.

**[Description of Drawings]**

FIGS. 1 to 4 illustrate NMR data of 1-O-acylceramide prepared according to an embodiment of the present disclosure.

FIG. 5 illustrates MS data of 1-O-acylceramide prepared according to an embodiment of the present disclosure.

FIG. 6 illustrates toxicity test data of 1-O-acylceramide prepared according to an embodiment of the present disclosure.

FIGS. 7 to 9 illustrate experimental data showing the effect of 1-O-acylceramide prepared according to an embodiment of the present disclosure on the expression of ceramide biosynthetic genes.

**[Mode for Invention]**

In one aspect, the present disclosure may relate to a 1-O-acylceramide having a structure of Chemical Formula 1 below, an isomer thereof, a salt thereof, a solvate thereof, or a hydrate thereof.

[Chemical Formula 1]

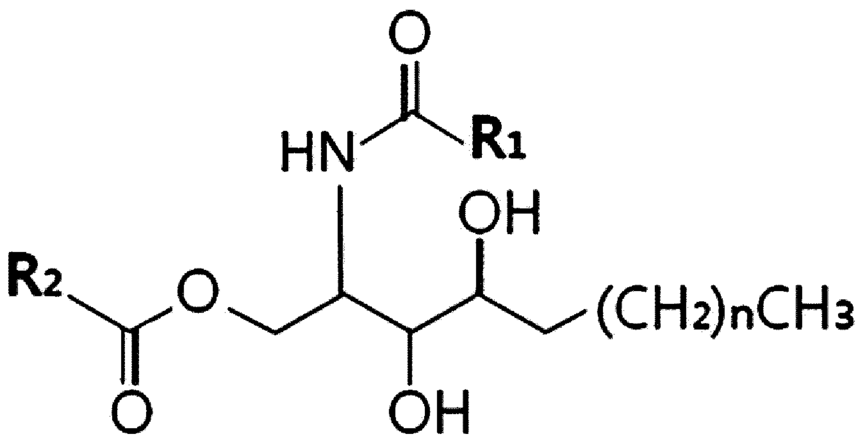

In the Chemical Formula 1, $R_1$ and $R_2$ are each independently a C9 to C31 saturated or unsaturated aliphatic chain, and n is an integer of 10 to 20.

In one embodiment, n in the Chemical Formula 1 may be 20 or less, 19 or less, 18 or less, 17 or less, 16 or less, 15 or less, 14 or less, or 13 or less. Also, in one embodiment, n in the Chemical Formula 1 may be 10 or more, 11 or more, or 12 or more. In one embodiment, n may be 12.

In one embodiment, the present disclosure may relate to a 1-O-acylceramide having a structure of Chemical Formula 2 below, an isomer thereof, a salt thereof, a solvate thereof, or a hydrate thereof.

[Chemical Formula 2]

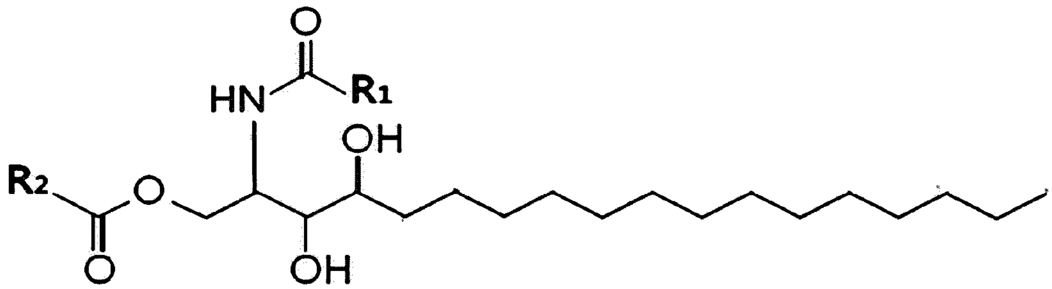

In the above Chemical Formula, $R_1$ and $R_2$ are each independently a C9 to C31 saturated or unsaturated aliphatic chain.

In one embodiment, the ceramide according to the present disclosure may use a ceramide of a phytosphingosine backbone, for example, N-stearoylphytosphingosine or N-oleanoylphytosphingosine. Until today, 1-O-acylceramides based on sphingosine (ceramide ENS) or sphinganine (ceramide ENDS) have been found in the skin, but 1-O-acylceramides based on phytosphingosine (ceramide ENP) have not been reported yet.

The 1-O-acylceramide has a structure in which a fatty acid is bonded to the hydroxyl group at position 1 of a head group, which has bidirectionally fatty acid chains, so it can spread fatty acids to both adjacent lamellar layers and plays a role in holding the lamellar layers on both sides. It has been reported that the 1-O-acylceramide accounts for about 5% of the skin barrier ceramide. The 1-O-acylceramide is a major component of skin barrier lamellae, and is a ceramide that acts as a linker or coupling bolt (coupling bolt-like ceramide) that binds lamella and neighboring lamella in both directions.

In one embodiment, $R_1$ and $R_2$ in the above Chemical Formula 1 may be unbranched linear saturated or unsaturated aliphatic chains. In one embodiment, $R_1$ may be an unsaturated aliphatic chain, and $R_2$ may be a saturated

aliphatic chain. In one embodiment, the carbon atoms of $R_1$ and $R_2$ may be C9 to C31. For example, the carbon atoms of $R_1$ and $R_2$ may be C9 or more, C11 or more, C13 or more, C15 or more, or C17 or more. Meanwhile, the carbon atoms of $R_1$ and $R_2$ may be, for example, C31 or less, C29 or less, C27 or less, C25 or less, C23 or less, C21 or less, C19 or less, or C17 or less. For example, the carbon atoms may be C15 to C19. For example, the carbon atoms of $R_1$ and $R_2$ may be C17. In one embodiment, $R_1$ may be a C17 unsaturated aliphatic chain, and $R_2$ may be a C17 saturated aliphatic chain. In one embodiment, $R_1COO$- may be oleoyl and $R_2COO$- may be stearyl.

In one embodiment, the fatty acid $R_1$ or $R_2$ may be used regardless of the number or presence of double bonds.

In one embodiment, the 1-O-acylceramide may be 1-O-acylceramide having a structure of Chemical Formula 3 below, an isomer thereof, a salt thereof, a solvate thereof, or a hydrate thereof.

[Chemical Formula 3]

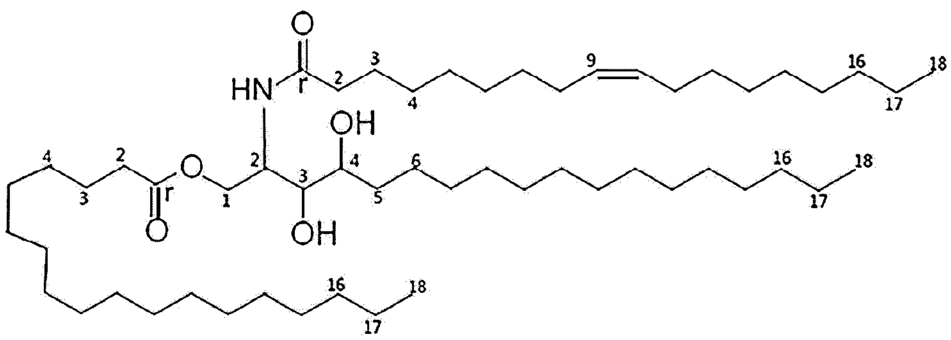

In one aspect, the present disclosure relates to a method for preparing 1-O-acylceramide, an isomer thereof, a salt thereof, a solvate thereof, or a hydrate thereof. In one embodiment, the 1-O-acylceramide may be a compound of the above Chemical Formula 1.

As used herein, "isomer" includes not only optical isomers (e.g., essentially pure enantiomers, essentially pure diastereomers or mixtures thereof) but also conformation isomers (i.e., isomers different only in angles of one or more chemical bonds), position isomers (especially, tautomers) or geometric isomers (i.e., cis-trans isomers).

As used herein, "essentially pure" means, for example, when used in connection with enantiomers or diastereomers, that the specific compound as an example of the enantiomer or the diastereomer is present in about 90% (w/w) or more, preferably about 95% or more, more preferably about 97% or more or about 98% or more, further more preferably about 99% or more, even more preferably about 99.5% or more.

In the present specification, the salt may be a pharmaceutically acceptable salt. As used herein, "pharmaceutically acceptable" means that use of a general medicinal dosage avoids a significant toxic effect and thus can be accepted or is accepted as appropriate for application to animals, particularly to human, by the government or a corresponding regulatory organization, or is listed in the pharmacopeia or regarded as being listed in general pharmacopeias.

As used herein, "pharmaceutically acceptable salt" refers to a salt according to one aspect of the present disclosure, which is pharmaceutically acceptable and exhibits the desired pharmacological activity of its parent compound.

As used herein, a "salt thereof or a "pharmaceutically acceptable salt" may include (1) an acid addition salt formed from an inorganic acid such as hydrochloric acid, hydrobromic acid, sulfuric acid, nitric acid, phosphoric acid, etc., or an organic acid such as acetic acid, propionic acid, hexanoic acid, cyclopentane propionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethane disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2,2,2]-oct-2-ene-1-carboxylic acid, glucoheptonic acid, 3-phenylpropionic acid, trimethylacetic acid, tert-butylacetic acid, lauryl sulfuric acid, gluconic acid, glutamic acid, hydroxynaphthoic acid, salicylic acid, stearic acid or muconic acid or (2) a salt formed as an acidic proton present in the parent compound is substituted.

As used herein, "hydrate" refers to a compound bound with water. It is used in a broad sense, including an inclusion compound which lacks chemical bonding with water.

As used herein, "solvate" refers to a higher-order compound formed between a solute molecule or ion, and a solvent molecule or ion.

In one embodiment, the preparation method may comprise binding a fatty acid to a ceramide of a phytosphingosine backbone.

In one embodiment, the step of binding the fatty acids may comprise adding and reacting at least one acyl donor of a free fatty acid, a triglyceride, and a fatty acid ester to the ceramide of the phytosphingosine backbone.

In one embodiment, the acyl donor may be the triglyceride. In addition, the triglyceride may be natural oil, natural butter, or wax including that derived from any of animals, plants, or minerals. In another aspect, the natural oil may be at least one oil, butter or wax selected from argan oil, macadamia nut oil, camellia oil, meadowfoam seed oil, rose hip oil, olive oil, moringa oil, baobab oil, green tea seed oil, red palm oil, horse oil, berry oil, sea buckthorn oil, sunflower seed oil, black sesame oil and emu oil, shea butter, soy bean oil, grape seed oil, black currant oil, avocado oil, beeswax, candelina wax, and carnauba wax.

Also, in one embodiment, the fatty acid ester may include methyl fatty acid ester, ethyl fatty acid ester, and the like.

In one embodiment, in the manufacturing method according to the present disclosure, as a method for binding the fatty acid to 1-OH of ceramide, the method disclosed in Korean Patent Registration No. 10-1816019 (Publication No. 10-2017-0084950) can be used. Alternatively, in one embodiment, an enzymatic conversion method may be used. For example, by using lipase produced from *Candida Antarctica* (E.C. 3.1.1), an acylation reaction can be induced at the 1-position hydroxyl group of ceramide.

In one embodiment, the manufacturing method according to the present disclosure may comprise mixing and melting the ceramide and the acyl donor; reacting the ceramide and the acyl donor melt.

In one embodiment, the manufacturing method according to the present disclosure may comprise mixing and melting the ceramide and the acyl donor; adding and reacting an enzyme to the ceramide and the acyl donor melt.

In one embodiment, the manufacturing method according to the present disclosure may be a method of using a solvent in the step of mixing and melting the ceramide and the acyl donor. In one embodiment, the solvent may be hexane.

In one embodiment, the manufacturing method according to the present disclosure may be a method that does not use a solvent in the step of mixing and melting the ceramide and the acyl donor.

In one embodiment, the manufacturing method according to the present disclosure may be a manufacturing method using a solvent in the step of reacting the acyl donor melt. In one embodiment, the solvent may be hexane.

In one embodiment, the manufacturing method according to the present disclosure may be a manufacturing method that does not use a solvent in the step of reacting the acyl donor melt.

In one embodiment, the step of binding the fatty acid may comprise adding the acyl donor to the ceramide of the phytosphingosine backbone, melting a mixture of the acyl donor and the ceramide by heating the mixture, and reacting the mixture for 12 hours or more. The heating for the melting and/or reaction may be 30 °C or more, 40 °C or more, 50 °C or more, 60 °C or more, 70 °C or more, 80 °C or more, 85 °C or more, 90 °C or more, 95 °C or more, 100 °C or more, 105 °C or more, or 110 °C or more. For example, the heating may be performed at temperature of 30 to 120 °C, or 80 to 120 °C.

In one embodiment, the step of binding the fatty acid may further comprise adding an enzyme after heating and melting the ceramide and the fatty acid. The enzyme may be a lipase. For example, the enzyme may be Lipozyme RM (Novozyme, Denmark, lipase (E.C. 3.1.1)).

In one embodiment, the reaction time may be 48 hours or less. For example, the reaction time may be 48 hours or less. 46 hours or less, 44 hours or less, 42 hours or less, 40 hours or less, 38 hours or less, 36 hours or less, 34 hours or less, 32 hours or less, 30 hours or less, 28 hours or less, 26 hours or less, 24 hours or less, 22 hours or less, 20 hours or less, 18 hours or less, or 16 hours or less. Meanwhile, the reaction time may be, for example, 10 minutes or more, 20 minutes or more, 30 minutes or more, 40 minutes or more, 50 minutes or more, 60 minutes or more, 80 minutes or more, 100 minutes or more, 2 hours or more, 4 hours or more, 6 hours or more, 8 hours or more, 10 hours or more, 12 hours or more, 14 hours or more, or 15 hours or more. In one embodiment, in the manufacturing method according to the present disclosure, the reaction time in the step of reacting the ceramide with the acyl donor melt may be 10 minutes to 48 hours.

In one embodiment, the reaction equivalent of the ceramide having the phytosphingosine backbone and the acyl donor may be 1: 1 to 3. For example, the reaction equivalent may be 1: 1 or more, 1: 1.1 or more, 1:1.2 or more, 1:1.3 or more, 1:1.4 or more, 1:1.5 or more, 1:1.6 or more, 1:1.7 or more, 1:1.8 or more, 1: 1.9 or more, or 1:2.0 or more. Alternatively, the reaction equivalent may be 1:3.0 or less, 1:2.9 or less, 1:2.8 or less, 1:2.7 or less, 1:2.6 or less, 1:2.5 or less, 1:2.4 or less, 1:2.3 or less, 1:2.2 or less, 1:2.1 or less, or 1:2.0 or less.

In one embodiment, the amount of the enzyme added may be 5 to 30 parts by weight when the weight of the substrate, sphingolipid, is 100 parts by weight.

In one embodiment, based on 100 parts by weight of the substrate, sphingolipid, the amount of the enzyme added may be 5 parts by weight or more, 6 parts by weight or more, 7 parts by weight or more, 8 parts by weight or more, 9 parts by weight or more, 10 parts by weight or more, 12 parts by weight or more, 14 parts by weight or more, 16 parts by weight or more, 18 parts by weight or more, 19 parts by weight or more, 20 parts by weight or more, 24 parts by weight or more, or 28 parts by weight or more. In another embodiment, based on 100 parts by weight of the substrate,

sphingolipid, the amount of the enzyme added may be 30 parts by weight or less, 28 parts by weight or less, 26 parts by weight or less, 24 parts by weight or less, 22 parts by weight or less, 20 parts by weight or less, 18 parts by weight or less, 16 parts by weight or less, 14 parts by weight or less, 13 parts by weight or less, 12 parts by weight or less, 11 parts by weight or less, or 10 parts by weight or less.

In one embodiment, the present disclosure may be a composition for reinforcing skin barrier function, comprising the 1-O-acylceramide, an isomer thereof, a salt thereof, a solvate thereof, or a hydrate thereof as an active ingredient.

In one embodiment, the present disclosure may relate to a use of the 1-O-acylceramide, the isomer thereof, the salt thereof, the solvate thereof or the hydrate thereof for preparing a composition for reinforcing skin barrier function. The use may be therapeutic or non-therapeutic.

In one embodiment, the present disclosure may be 1-O-acylceramide, an isomer thereof, a salt thereof, a solvate thereof, or a hydrate thereof for reinforcing skin barrier function. The 1-O-acylceramide may be the above-described 1-O-acylceramide.

In one embodiment, the present disclosure may relate to a method for reinforcing skin barrier function, comprising administering 1-O-acylceramide, an isomer thereof, a salt thereof, a solvate thereof or a hydrate thereof to a subject in need thereof. The 1-O-acylceramide may be the above-described 1-O-acylceramide.

In one embodiment, the composition may be a pharmaceutical composition, a cosmetic composition or a food composition.

In one embodiment, the 1-O-acylceramide, the isomer thereof, the salt thereof, the solvate thereof, or the hydrate thereof may be used for treatment, alleviation, or prevention of skin diseases caused by reduction in skin barrier function. For example, the skin diseases caused by the reduction in skin barrier function include atopy or psoriasis.

In one embodiment, the 1-O-acylceramide, the isomer thereof, the salt thereof, a solvate thereof, or the hydrate thereof may be used for recovering or improving the skin barrier function damaged by various causes such as disease, autoimmunity or external stimulation. For example, it can be used for the purpose of recovering or improving the skin barrier function damaged by diseases such as contact dermatitis, allergic dermatitis, or acne, ultraviolet rays, or fine dust.

In one embodiment, the 1-O-acylceramide, an isomer thereof, a salt thereof, a solvate thereof, or a hydrate thereof may be administered to a subject in need thereof at a daily dose of 0.09 mg/kg to 1.10 mg/kg. For example, the daily dose of the 1-O-acylceramide, an isomer thereof, a salt thereof, a solvate thereof or a hydrate thereof may be 0.09 mg/kg or more, 0.10 mg/kg or more, 0.12 mg/kg or more, 0.14 mg/kg or more, 0.16 mg/kg or more, 0.18 mg/kg or more, 0.20 mg/kg or more, 0.22 mg/kg or more, 0.24 mg/kg, 0.26 mg/kg or more, 0.28 mg/kg or more, 0.30 mg/kg or more, 0.32 mg/kg or more, 0.34 mg/kg or more, 0.36 mg/kg or more, 0.38 mg/kg or more, 0.40 mg/kg or more, 0.42 mg/kg, or 0.44 mg/kg or more. Alternatively, the daily dose of the 1-O-acylceramide, an isomer thereof, a salt thereof, a solvate thereof or a hydrate thereof may be 1.11 mg/kg or less, 1.10 mg/kg or less, 1.05 mg/kg or less, 1.00 mg/kg or less, 0.90 mg/kg or less, 0.80 mg/kg or less, 0.70 mg/kg or less, 0.60 mg/kg or less, 0.50 mg/kg or less, 0.49 mg/kg or less, 0.48 mg/kg or less, 0.47 mg/kg or less, or 0.46 mg/kg or less.

One aspect of the present disclosure provides a cosmetic composition comprising 1-O-acylceramide, an isomer thereof, a salt thereof, a solvate thereof, or a hydrate thereof. The cosmetic composition comprises a cosmetically or dermatologically acceptable medium or substrate. The composition may be provided in the form of any formulation suitable for topical application. For example, the composition may be used in the form of solutions, gels, solids, paste anhydrides, oil-in-water emulsions, water-in-oil emulsions, multi emulsions, suspensions, microemulsions, microcapsules, microgranules, ionic (liposome) and nonionic vesicular dispersants, foam, aerosol additionally containing a compressed propellant, or patches. The composition may be formulated according the methods commonly employed in the art.

The cosmetic composition may further comprise, in addition to the above-described substances, other ingredients that may provide a synergic effect to the main effect within a range not negatively affecting the main effect. The other ingredients may be selected and combined by those skilled in the art without difficulty depending on the formulation type or purpose of use. For example, the cosmetic composition of the present disclosure may comprise, in addition to the active ingredient, other ingredients commonly mixed in a cosmetic composition as needed. Examples include an oil and fat, a humectant, an emollient, a surfactant, an organic or inorganic pigment, an organic powder, a UV absorbent, a preservative, a sterilizer, an antioxidant, a stabilizer, a thickener, glycerin, a pH control agent, an alcohol, a pigment, a fragrance, a blood circulation accelerator, a coolant, an antiperspirant, purified water, etc. However, the other ingredients that may be comprised in the cosmetic composition are not limited thereto and the amount thereof may be determined within a range not negatively affecting the purpose and effect of the present disclosure.

The formulation type of the cosmetic composition is not particularly limited and may be selected adequately depending on purposes. For example, it may be prepared into one or more formulation selected from a group consisting of a soap-type formulation, a softening lotion, a nourishing lotion, an essence, a nourishing cream, a massage cream, a pack, a gel, a makeup base, a foundation, a powder, a lipstick, a patch, a spray, an eye cream, an eye essence, a cleansing cream, a cleansing foam, a cleansing water, a cleanser, a hair shampoo, a hair conditioner, a hair treatment product, a hair essence, a hair lotion, a scalp and hair tonic, a scalp essence, a hair gel, a hair spray, a hair pack, a body lotion, a body cream, a body oil and a body essence, although not being limited thereto.

One aspect of the present disclosure provides a pharmaceutical composition comprising 1-O-acylceramide, an isomer thereof, a salt thereof, a solvate thereof, or a hydrate thereof. The pharmaceutical composition may further comprise, in addition to the active ingredient, pharmaceutical adjuvants or other therapeutically useful substances such as a preservative, a stabilizer, a wetting agent, an emulsifier, a salt and/or buffer for control of osmotic pressure, a diluent (e.g., lactose, dextrose, sucrose, mannitol, sorbitol, cellulose or glycine), a lubricant (e.g., silica, talc, stearic acid and a magnesium or calcium salt thereof or polyethylene glycol), a binder (e.g., magnesium aluminum silicate, starch paste, gelatin, tragacanth, methyl cellulose, sodium carboxymethyl cellulose or polyvinylpyrrolidone), etc. In some occasions, it may further comprise other pharmaceutical additives such as a disintegrant, e.g., starch, agar, alginic acid or a sodium salt thereof, an absorbent, a colorant, a flavor, a sweetener, etc.

The pharmaceutical composition may be prepared into formulations for various oral or parenteral administration forms according to commonly employed methods. Formulations for the parenteral administration may include, for example, a drop, an ointment, a lotion, a gel, a cream, a spray, a suspension, an emulsion, a suppository, a patch, an injection, etc., although not being limited thereto.

The pharmaceutical composition according to an aspect of the present disclosure may be administered orally or parenterally, such as transdermally or intravenously. The composition is prepared to be suitable for each formulation and standards. In particular, a composition for intravenous injection is prepared with very high purity by excluding any unsuitable additive.

An administration dosage of the active ingredient will vary depending on the age, gender and body weight of a subject to be treated, disease, pathological condition, administration route or the discretion of a diagnoser. The determination of the administration dosage considering these factors is within the level of those skilled in the art.

**[Mode for implementation of the disclosure]**

Hereinafter, the present disclosure will be described in detail through examples, comparative examples and experimental examples. However, the examples, comparative examples and experimental examples are for illustrative purposes only and it will be apparent to those of ordinary skill in the art that the scope of the present disclosure is not limited by the following examples, comparative examples and experimental examples.

Example 1) Reaction between sphingolipids and acyl donors

As shown in the table below, 1 equivalent mole of an acyl donor was added to a certain amount of sphingolipids, the mixture was heated to 100 to 110 °C to melt the mixture, and then reacted for 24 hours to obtain a new ceramide reactant. The conversion rate to 1-O-acylceramide was calculated by analyzing the new ceramide reactant obtained by the reaction and calculating the content of 1-O-acylceramide. As shown in the table below, in the absence of enzymes, the conversion rate to 1-O-acylceramide was as low as a level of 5 to 10 %. There was no significant difference in the conversion rate between the case of using fatty acid as an acyl donor and the case of using triglyceride form as an acryl donor.

[Table 1]

| Sphingolipid | Acyl donor | Temperature (°C) | Conversion rate (%) |
|---|---|---|---|
| N-oleoylphytosphingosine | Stearic acid | 100 | 5 |
| | Tri stearin | 105 | 5 |
| Complex ceramide NP (C16 to C24 ceramide NP) | Stearic acid | 100 | 10 |
| Phytosphingosine | Stearic acid | 100 | 5 |
| | Tri stearin | 105 | 5 |

Example 2) The reaction between the sphingolipid and the acyl donor by the enzyme

As shown in the table below. 1 equivalent mole of each acyl donor was added to oleoylphytosphingosine (N-oleoylphytosphingosine, ceramide NP) and heated to 90 °C to melt the mixture. Then, Lipozyme RM (Novozyme, Denmark, lipase (E.C. 3.1.1)) was added in an amount of 5 parts by weight based on 100 parts by weight of sphingolipid, and then reacted for 24 hours to confirm the conversion rate. As shown in the table, there was a difference in the conversion rate to 1-O-acylceramide depending on the acyl donor, and the acyl donor in the form of triglyceride showed the highest conversion rate of 65 % based on 24 hours.

[Table 2]

| Sphingolipid | Acyl donor | Conversion rate (%) |
|---|---|---|
| Oleoylphytosphingosine | Methyl stearate | 27 |
| | Ethyl stearate | 36 |
| | Stearic acid | 35 |
| | Tri stearin | 65 |

Example 3) The conversion rate according to the effect of the solvent

As shown in figures. 1.5 equivalent mole of tristearin in the form of triglyceride was added as an acyl donor to a certain amount of oleoylphytosphingosine (N-oleoylphytosphingosine, ceramide NP) and 2.5-fold volume of solvent was added to heat and melt the mixture. Then, Lipozyme RM (Novozyme, Denmark, lipase (E.C. 3.1.1)) was added in an amount of 5 parts by weight based on 100 parts by weight of the sphingolipid, and then reacted for 24 hours to confirm the conversion rate. As shown in the table below, the case where the solvent was added showed lower conversion to 1-O-acylceramide on a 24-hour basis, compared to the case where the solvent was not added. In some cases, the reaction hardly occurred depending on the type of solvents.

[Table 3]

| Sphingolipid | Acyl donor | Solvent | Amount of solvent | Temperat ure (°C) | Conversion rate (%) |
|---|---|---|---|---|---|
| Oleoylphytosphingosine | Tri stearin | | | 90 | 63 |
| | Tri stearin | tert-amyl alcohol | 2.5-fold | 90 | 2 |
| | Tri stearin | Hexane | 2.5-fold | 55 | 56 |

Example 4) The reaction rate change according to the reaction time

As shown in the table below. 1.5 equivalent mole of an acyl donor was added to a certain amount of oleoylphytosphingosine (N-oleoylphytosphingosine, ceramide NP), dissolved in 10-fold volume of hexane, and then Lipozyme RM (Novozyme, Denmark, lipase (E.C. 3.1.1)) was added in an amount of 5 parts by weight based on 100 parts by weight of sphingolipid, and then reacted, thereby confirming the conversion rate over time. In the case of tristearin, the reaction proceeded after 16 hours, and the conversion rate hardly changed, whereas in the case of stearic acid, the conversion rate decreased with the passage of time. Therefore, it was found that the reaction proceeded further after the formation of 1-O-acylceramide to produce side reactants.

[Table 4]

| Sphingolipid | Acyl donor | Reaction time (hr) | Conversion rate (%) |
|---|---|---|---|
| Oleoylphytosphingosine | Tri stearin | 16 | 53 |
| | | 24 | 56 |
| | | 48 | 56 |
| | | 72 | 58 |
| | Stearic acid | 16 | 62 |
| | | 24 | 63 |
| | | 48 | 58 |
| | | 72 | 54 |

Example 5) The effect of the amount of the acyl donor

As shown in the table below. The acyl donor in the amount shown in the table below was added to a certain amount of oleoylphytosphingosine (N-oleoylphytosphingosine, ceramide NP), and dissolved in 10-fold volume of hexane. Then, Lipozyme RM (Novozyme, Denmark, lipase (E.C. 3.1.1)) was added in an amount of 5 parts by weight based on 100 parts by weight of sphingolipid, and then reacted for 24 hours to confirm the conversion rate. As shown in the table below, it was found that the conversion rate of 1-O-acylceramide increased as the amount of acyl donor increased.

[Table 5]

| Sphingolipid | Acyl donor | Amount (equivalent) of acyl donor | Conversion rate (%) |
|---|---|---|---|
| Oleoylphytosphingosine | Tri stearin | 1 | 45 |
| | | 1.5 | 56 |
| | | 2 | 69 |
| | | 2.5 | 68 |

Example 6) The conversion rate test according to the amount of enzyme As shown in the table below. 1.5 equivalent of an acyl donor was added to a certain amount of oleoylphytosphingosine (N-oleoylphytosphingosine, ceramide NP) and stearoylphytosphingosine (N-stearoylphytosphingosine, ceramide NP) and then dissolved in 10-fold volume of Hexane. Then, Lipozyme RM (Novozyme, Denmark, lipase (E.C. 3.1.1)) was added, heated to 90 °C, reacted for 1 hour, thereby confirming the conversion rate of 1-O-acylceramide. As shown in the table below, while the amount of enzymes increased up to 10 parts by weight based on 100 parts by weight of the substrate, sphingolipid, the conversion rate of 1-O-acylceramide increased, but at the higher enzyme concentrations, the increase in 1-O-acylceramide did not increase significantly.

[Table 6]

| Sphingolipid | Acyl donor | Amount of enzymes(parts by weight) | Conversion rate (%) |
|---|---|---|---|
| N-oleoylphytosphingosine | Stearic acid | 5 | 63 |
| | | 10 | 74 |
| | | 20 | 76 |
| N-stearoylphytosphingosine | Stearic acid | 5 | 48 |
| | | 10 | 68 |
| | | 20 | 76 |

Example 7) Structural analysis of 1-O-acylceramide

The 1-O-acylceramide obtained from the reaction was separated and purified, and the molecular weight was confirmed by a mass spectrometry (MS) (MW: 882.82), and it was confirmed by NMR that the acyl chain was bound to the hydroxyl group at position 1 (FIGS. 1 to 5).

Example 8) Analysis of the effect of 1-O-acylceramide on ceramide biosynthesis

Cytotoxicity test

Human Epidermal Keratinocyte (neonatal (HEKn), Thermo Fisher Scientific, USA) was placed in $7\times10^3$ cells in a 96-well plate and cultured for 24 hours at 37 °C under 5% $CO_2$ aeration conditions. Then, the 1-O-acylceramide sample was treated so that the final volume was 100ul. The 96-well plate treated with 1-O-acylceramide was cultured for an additional 24 hours at 37 °C under 5% $CO_2$ aeration conditions. After removing the medium, it was washed once with phosphate buffered saline (PBS) and the same amount of 3-(4,5-dimethylthiazol-2-yl)-2,5-diphenyltetrazolium bromide (MTT) solution was added. It was incubated at 37° C for 90 minutes. After removing the MTT solution, 100ul of stop solution (dimethyl sulfoxide) was added and left at room temperature for 20 minutes. The absorbance was measured at 570 nm, and the results were shown in FIG. 6 .

As shown in FIG. 6, 1-C-acylceramide was toxic to cells depending on its concentration. This was generally found to be similar to the range of toxicity exhibited by ceramide or phytosphingosine.

Cell culture and RNA extraction

Human epidermal keratinocytes (neonatal (HEKn), Thermo Fisher Scientific, USA) were added to 12-well plate, $1\times10^5$ cells per well and cultured for 24 hours at 37°C under 5% $CO_2$ aeration conditions. After treatment with the 1-O-acylcermide prepared in the above Example at concentrations of 0.1, 0.5, and 1uM, respectively, the cells were additionally incubated for 24 hours at 37 °C under 5% $CO_2$ aeration conditions. After removing the medium and washing once with PBS buffer, RNA was extracted using NucleoSpin RNA Plus kit (Germany). As for the conditions, 350ul LBP (lysate buffer) was put into each well, scraped with a scraper, and cell lysate was collected on the NucleoSpin gDNA Removal Column (yellow ring), and then centrifuged at 11,000g for 30 seconds. 100ul binding solution (BS) was added to the collected solution, mixed, transferred to NucleoSpin RNA Plus Column (blue ring), and centrifuged at 11,000g for 15 seconds. 200ul Buffer WB1 (washing buffer) was added to the column, washed by centrifugation at 11,000g for 15 seconds, and 600ul Buffer WB2 (washing buffer) was added and washed by centrifugation at 11,000g for 15 seconds. Finally, 250ul buffer WB2 (washing buffer) was added and washed by centrifugation at 11,000g for 2 minutes. Each step above is to remove remaining proteins, lipids and DNA. 20ul RNase-free $H_2O$ was added and centrifuged at 11,000g for 1 minute (2 times). The extracted RNAs were collected in a sterilized microtube and the RNAs were quantified. The RNAs were measured for absorbance at 260 nm and 280 nm, and the amount of RNAs was calculated. Here, RNase free water was used as a blank.

Synthesis of cDNA with RNA

1ul of oligo-dT primer was added to RNA calibration value + DEPC water calibration value (total 11ul), collected by centrifugation, and heated at 65 °C for 5 minutes. After centrifugation while cooling, buffer 4ul + dNTP mix 2ul + RNase inhibitor 1ul + RNA transcriptase 1ul (total 20ul) were added to each sample, left at room temperature for 5 minutes, and then enzymatically reacted at 42 °C for 1 hour. The enzyme stop reaction was performed at 70 °C for 5 minutes.

RT-PCR

The RT-PCR mixture was as follows; a total volume of 25 ul was prepared by 2 ul of synthesized cDNA + 0.5 ul of primer mix (10 uM) + 12.5 ul of TB Green® Premix Ex Taq with distilled water filling the remaining volume. As operating conditions, after heating once at 95°C-1 second, 95°C-5 seconds and 60°C-1 second were cycled 40 times. The primer sequences used in this case were shown in Table 7. The values obtained through RT-PCR were quantified using Equations (1) and (2) and then quantitatively analyzed.

$$\Delta C_q = C_{q\,(Target\ Gene)} - C_{q\,(GAPDH)} \cdots\cdots\cdots \text{equation}(1)$$

$$\Delta C_q\ \text{Expression} = 2^{-\Delta C_q} \cdots\cdots\cdots \text{equation}(2)$$

[Table 7]

| Name | sequence | Sequence Listing No. |
|---|---|---|
| GAPDH (F) | GAGTCAACGGATTTGGTCGT | SEO ID NO: 1 |
| GAPDH (R) | TTGATTTTGGAGGGATCTCG | SEO ID NO: 2 |
| SPTLC-1 (F) | GCGCGCTACTTGGAGAAAGA | SEO ID NO: 3 |
| SPTLC-1 (R) | TGTTCCACCGTGACCACAAC | SEO ID NO: 4 |
| SPTLC-2 (F) | AGCCGCCAAAGTCCTTGAG | SEO ID NO: 5 |
| SPTLC-2 (R) | CTTGTCCAGGTTTCCAATTTCC | SEO ID NO: 6 |

(continued)

| Name | sequence | Sequence Listing No. |
|---|---|---|
| CER3 (F) | ACATTCCACAAGGCAACCATTG | SEO ID NO: 7 |
| CER3 (R) | CTCTTGATTCCGCCGACTCC | SEO ID NO: 8 |

SPTLC-1 and SPTLC-2, the primer sequences used, are serine-palmitoyltransferases that correspond to the rate limiting step in the ceramide biosynthetic pathway, and the condensation reaction between serine and palmitoyl CoA occurs by this enzyme, resulting in the synthesis of the sphingoid backbone. As a result, a series of biosynthetic reactions in which ceramide is synthesized proceeds. Through this example, it is expected that 1-O-acylceramide can play a role in maintaining the homeostasis of the skin barrier by promoting the de novo synthesis of ceramide in skin cells by increasing the expression of serine-palmitoyltransferase by about 2 times.

In addition, as the activity of ceramide synthase 3 involved in ceramide synthesis is increased, it is expected to have an effect of promoting ceramide synthesis in the skin.

**Sequence Listing Free Text**

| Name sequence | Sequence Listing No. | |
|---|---|---|
| GAPDH (F) | GAGTCAACGGATTTGGTCGT | SEO ID NO: 1 |
| GAPDH (R) | TTGATTTTGGAGGGATCTCG | SEO ID NO: 2 |
| SPTLC-1 (F) | GCGCGCTACTTGGAGAAAGA | SEO ID NO: 3 |
| SPTLC-1 (R) | TGTTCCACCGTGACCACAAC | SEO ID NO: 4 |
| SPTLC-2 (F) | AGCCGCCAAAGTCCTTGAG | SEO ID NO: 5 |
| SPTLC-2 (R) | CTTGTCCAGGTTTCCAATTTCC | SEO ID NO: 6 |
| CER3 (F) | ACATTCCACAAGGCAACCATTG | SEO ID NO: 7 |
| CER3 (R) | CTCTTGATTCCGCCGACTCC | SEO ID NO: 8 |

## Claims

**1.** 1-O-acylceramide having a structure of Chemical Formula 1, an isomer thereof, a salt thereof, a solvate thereof, or a hydrate thereof:

[Chemical Formula 1]

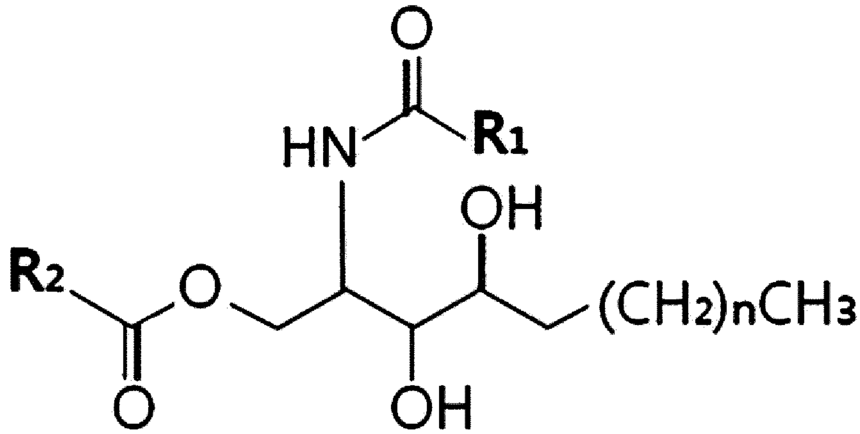

wherein in the Chemical Formula 1, $R_1$ and $R_2$ are each independently a C9 to C31 saturated or unsaturated aliphatic chain, and n is an integer of 10 to 20.

2. The 1-O-acylceramide, the isomer thereof, the salt thereof, the solvate thereof, or the hydrate thereof of claim 1, wherein the $R_1$ and the $R_2$ are unbranched linear saturated or unsaturated aliphatic chains.

3. The 1-O-acylceramide, the isomer thereof, the salt thereof, the solvate thereof, or the hydrate thereof of claim 2, wherein the $R_1$ and the $R_2$ have a carbon number of C13 to C21.

4. The 1-O-acylceramide, the isomer thereof, the salt thereof, the solvate thereof, or the hydrate thereof of claim 3, wherein the 1-O-acylceramide has a structure of Chemical Formula 3:

[Chemical Formula 3]

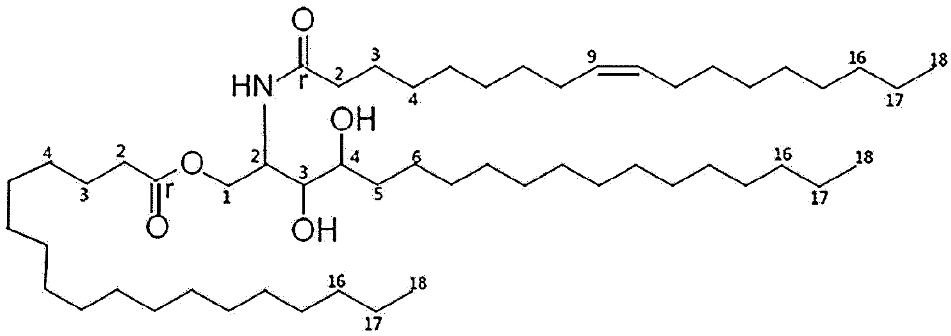

5. A method for preparing the 1-O-acylceramide, the isomer thereof, the salt thereof, the solvate thereof, or the hydrate thereof of any one of claims 1 to 4, comprising:
binding a fatty acid to a ceramide having a phytosphingosine backbone.

6. The method of claim 5, wherein the binding the fatty acid comprises adding at least one acyl donor selected from the group consisting of a free fatty acid, a triglyceride and a fatty acid ester to the ceramide having a phytosphingosine backbone to react the acyl donor with the ceramide having a phytosphingosine backbone.

7. The method of claim 6, wherein the binding the fatty acid comprises adding the acyl donor to the ceramide having the phytosphingosine backbone, melting a mixture of the acryl donor and the ceramide by heating the mixture, and reacting the mixture for 12 hours or more.

8. The method of claim 6, wherein the binding the fatty acid further comprises, after adding the acyl donor to the ceramide having the phytosphingosine backbone and melting a resulted mixture of the ceramide and the acyl donor by heating, adding an enzyme to the resulted melted mixture of the ceramide and the acyl donor.

9. The method of claim 7, wherein the acyl donor is triglyceride.

10. The method of claim 6, wherein the reaction is performed without adding a solvent or with adding hexane.

11. The method of claim 7, wherein time for the reaction is 40 hours or less.

12. The method of claim 6, wherein a reaction equivalent of the ceramide having the phytosphingosine backbone and the acyl donor is 1: 1 to 2.5.

13. The method of claim 8, wherein an amount of the enzyme added is 5 to 30 parts by weight based on 100 parts by weight of a sphingolipid, which is a substrate.

14. A composition for reinforcing skin barrier function, comprising the 1-O-acylceramide, the isomer thereof, the salt thereof, the solvate thereof, or the hydrate thereof of any one of claims 1 to 4 as an active ingredient.

15. The composition of claim 14, wherein the composition is a cosmetic composition for reinforcing skin barrier function.

16. The composition of claim 14, wherein the composition is a food composition for reinforcing skin barrier function.

**17.** The composition of claim 14, wherein the composition is a pharmaceutical composition for treating, alleviating or preventing a skin disease caused by reduction in skin barrier function.

**18.** The composition of claim 14, wherein the 1-O-acylceramide, the isomer thereof, the salt thereof, the solvate thereof, the hydrate thereof is administered at a daily dose of 0.09 mg/kg to 1.10 mg/kg.

[FIG. 1]

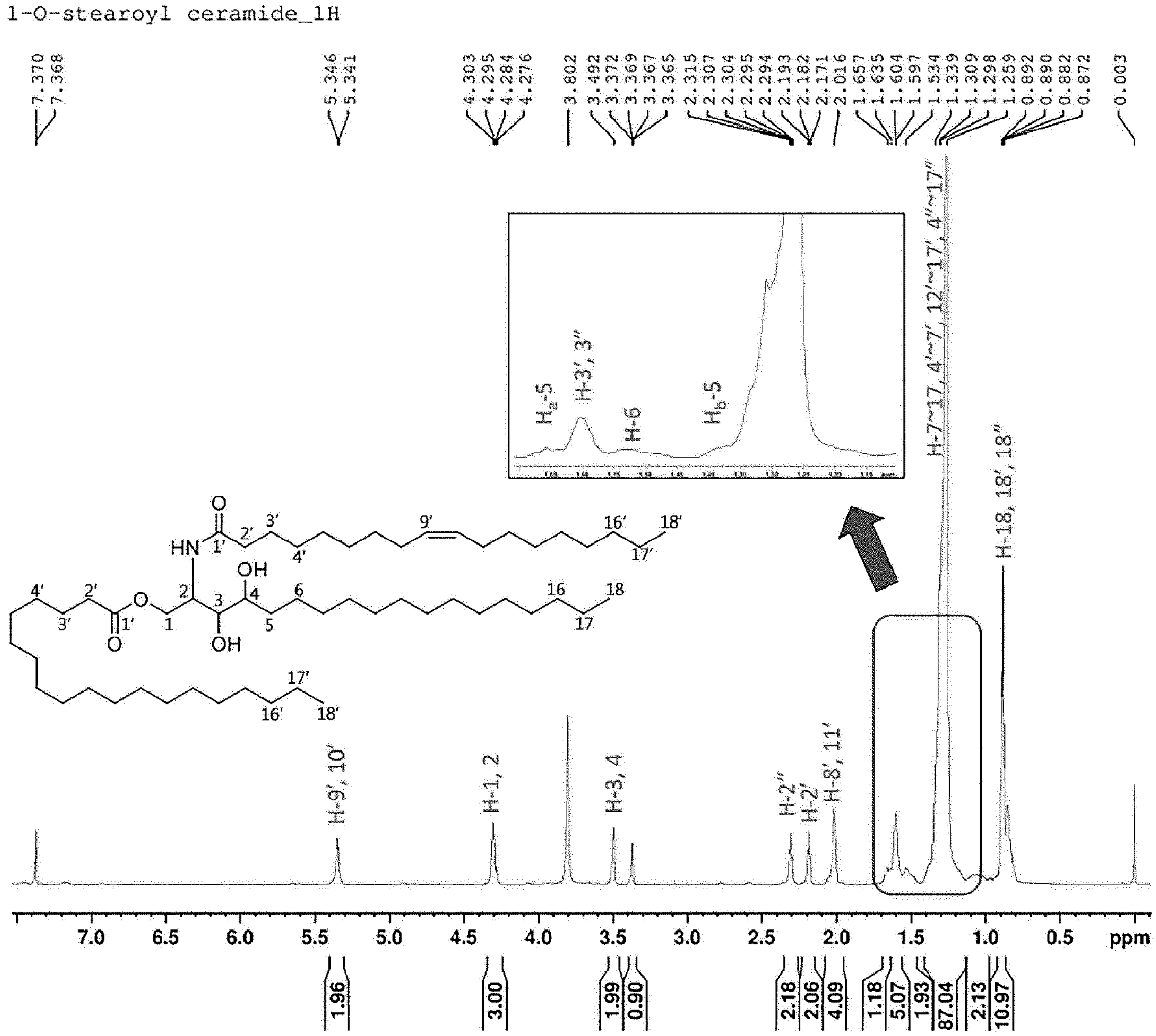
1-O-stearoyl ceramide_1H
7.370
7.368
5.346
5.341
4.303
4.295
4.284
4.276
3.802
3.492
3.372
3.369
3.367
3.365
2.315
2.307
2.304
2.295
2.294
2.193
2.182
2.171
2.016
1.657
1.635
1.604
1.597
1.534
1.339
1.309
1.298
1.259
0.892
0.890
0.882
0.872
0.003
H-9′, 10′
H-1, 2
H-3, 4
H-2″
H-2′
H-8′, 11′
H-7~17, 4′~7′, 12′~17′, 4″~17″
H-18, 18′, 18″
$H_a$-5
H-3′, 3″
H-6
$H_b$-5
ppm
1.96
3.00
1.99
0.90
2.18
2.06
4.09
1.18
5.07
1.93
87.04
2.13
10.97

[FIG. 2]

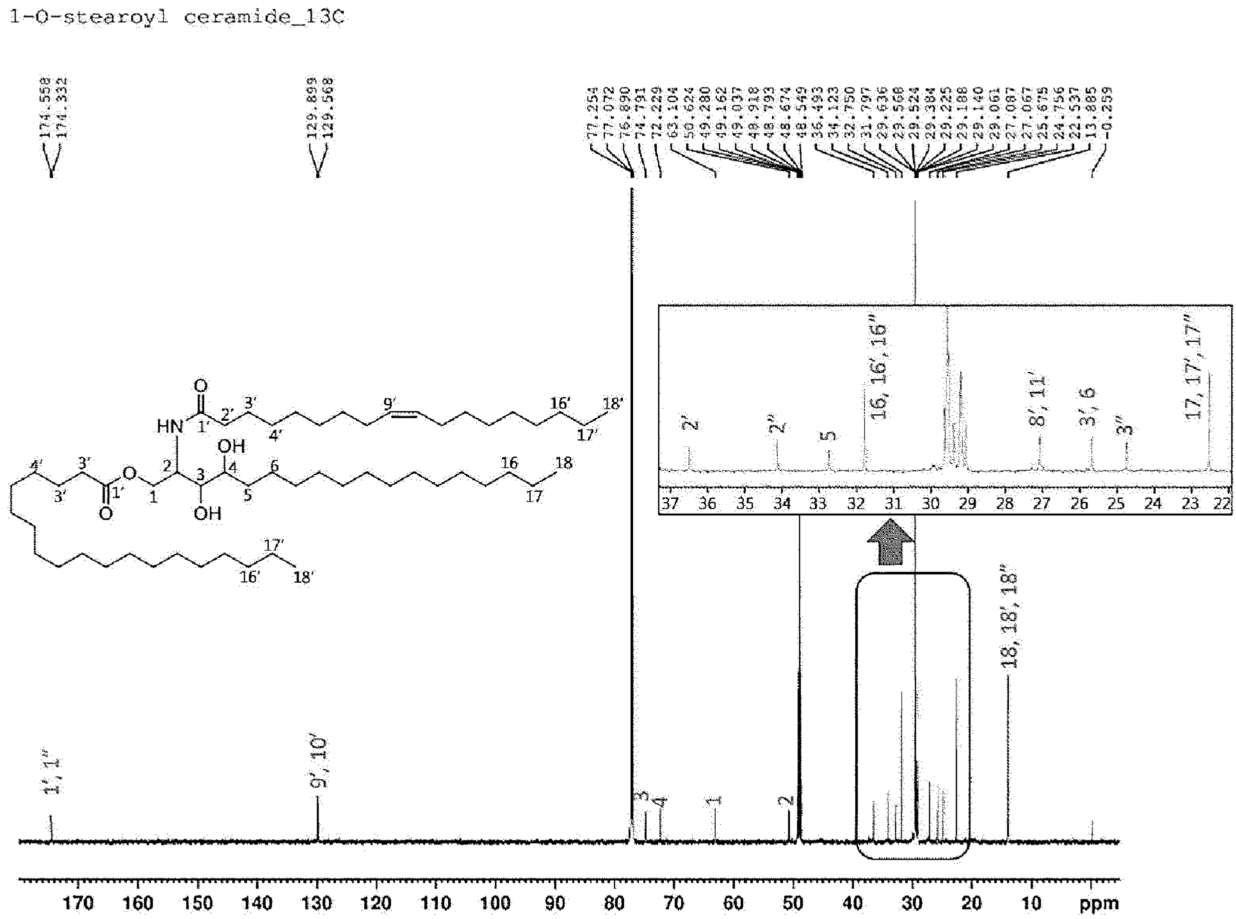
1-O-stearoyl ceramide_13C
1', 1"
9', 10'
3
4
1
2
18, 18', 18"
2'
2"
5
16, 16', 16"
8', 11'
3', 6
3"
17, 17', 17"
ppm

[FIG. 3]

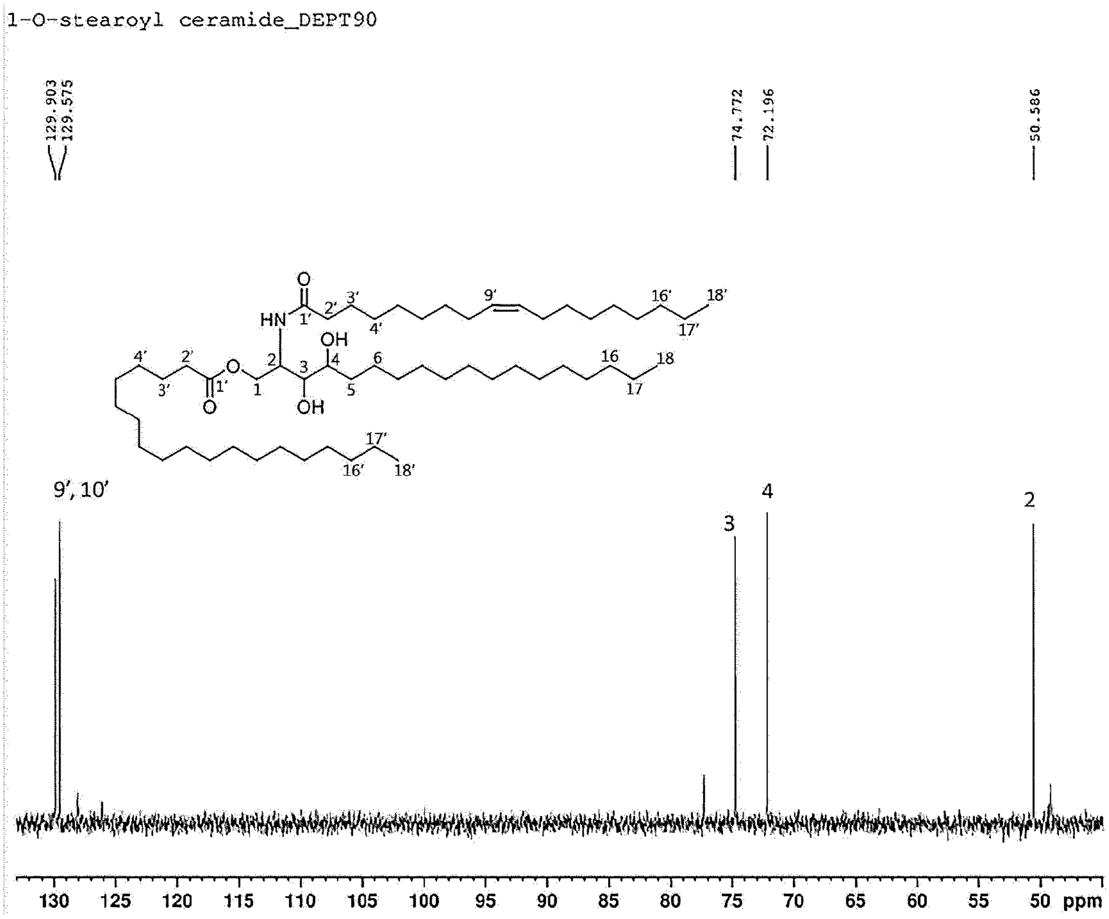
1-O-stearoyl ceramide_DEPT90
129.903
129.575
74.772
72.196
50.586
9', 10'
3
4
2
130 125 120 115 110 105 100 95 90 85 80 75 70 65 60 55 50 ppm

[FIG. 4]

1-O-stearoyl ceramide_DEPT135

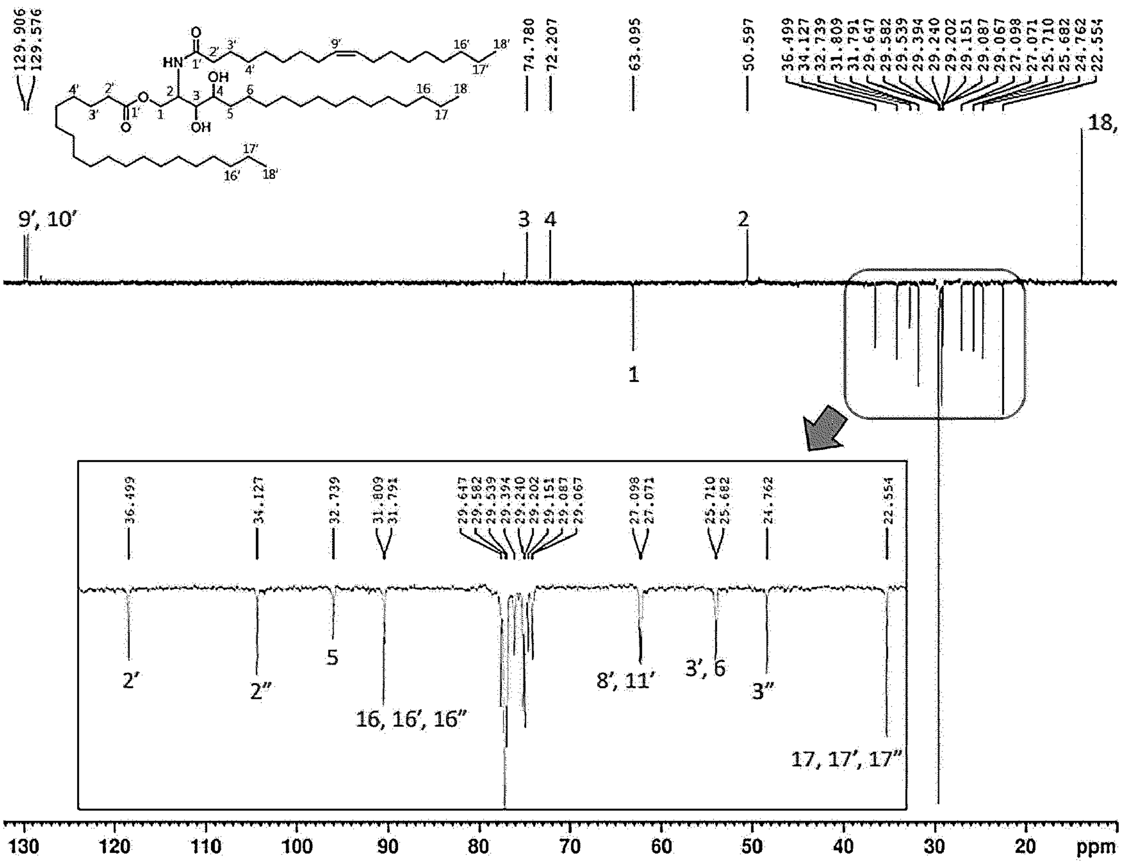

[FIG. 5]

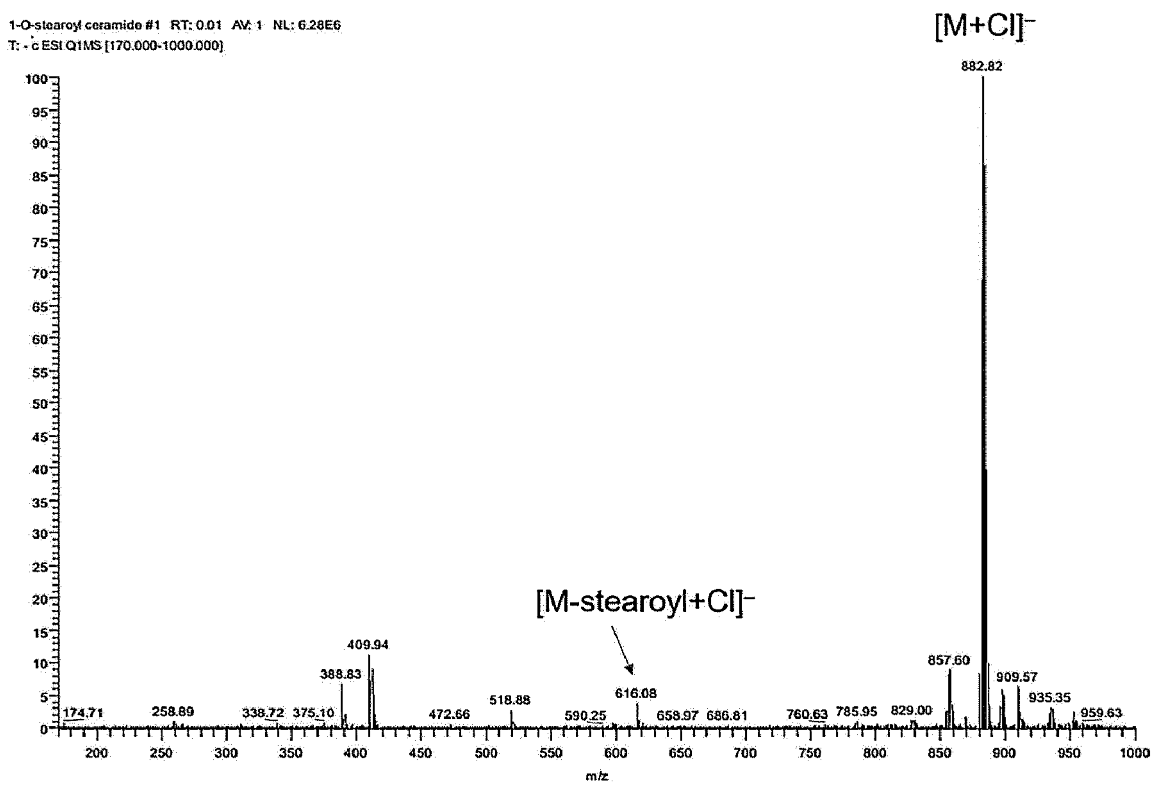
1-O-stearoyl ceramide #1 RT: 0.01 AV: 1 NL: 6.28E6
T: - c ESI Q1MS [170.000-1000.000]
[M+Cl]⁻
882.82
[M-stearoyl+Cl]⁻
409.94
388.83
518.88
616.08
857.60
909.57
935.35
174.71
258.89
338.72
375.10
472.66
590.25
658.97
686.81
760.63
785.95
829.00
959.63
m/z

[FIG. 6]

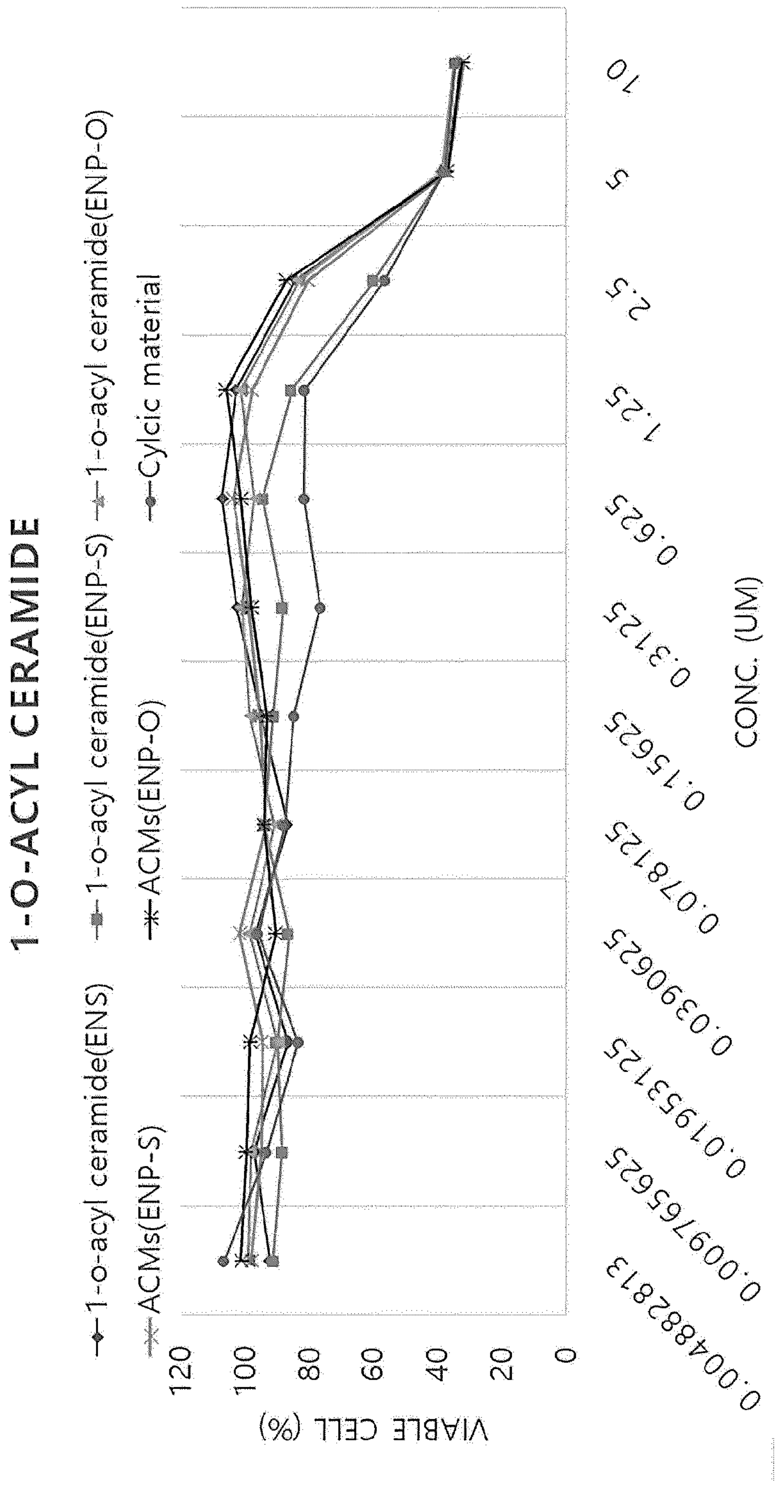
1-O-ACYL CERAMIDE
1-o-acyl ceramide(ENS)
1-o-acyl ceramide(ENP-S)
1-o-acyl ceramide(ENP-O)
ACMs(ENP-S)
ACMs(ENP-O)
Cylcic material
VIABLE CELL (%)
0
20
40
60
80
100
120
CONC. (UM)
10
5
2.5
1.25
0.625
0.3125
0.15625
0.078125
0.0390625
0.01953125
0.009765625
0.004882813

[FIG. 7]

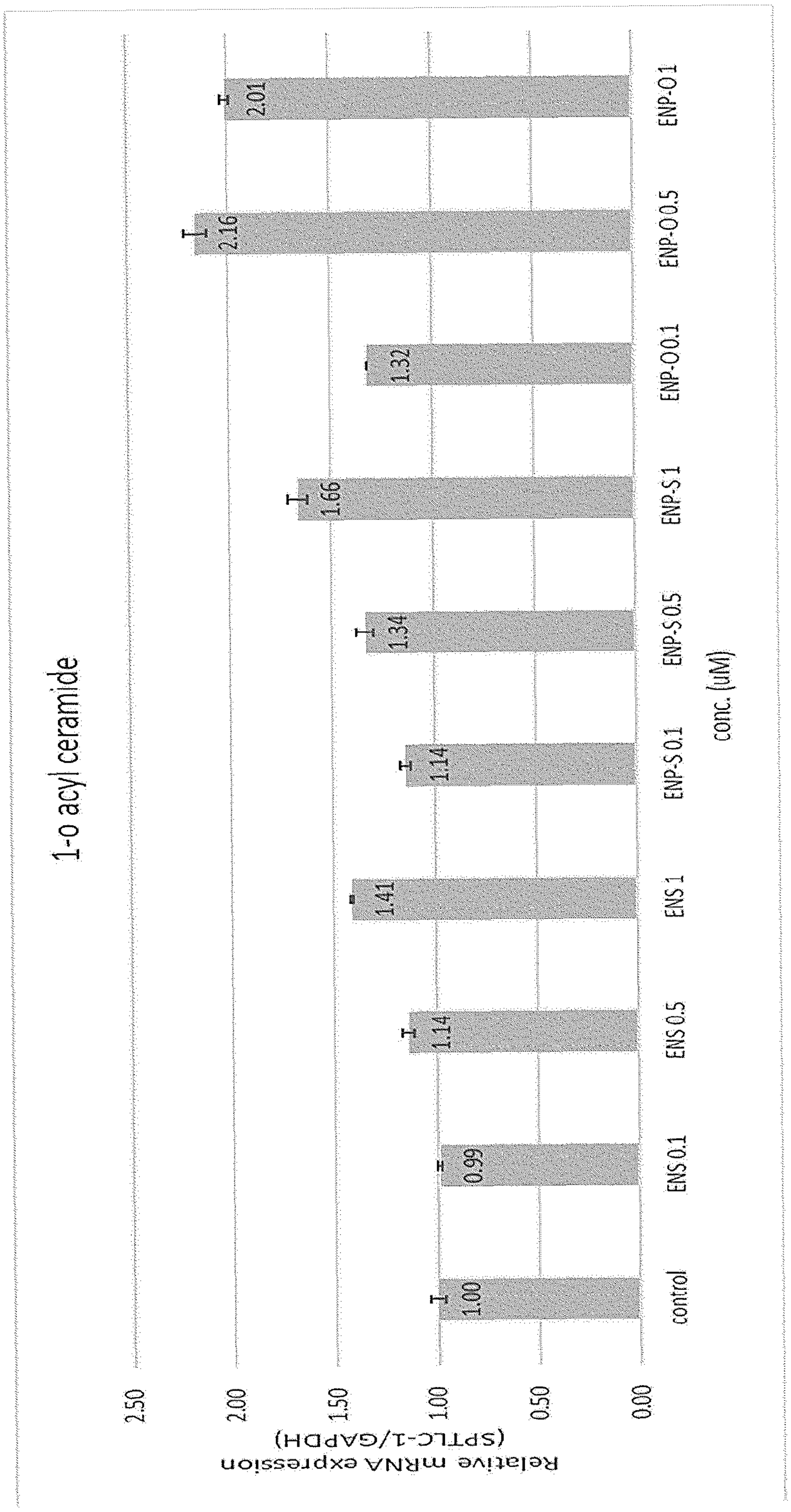
1-o acyl ceramide
Relative mRNA expression
(SPTLC-1/GAPDH)
2.50
2.00
1.50
1.00
0.50
0.00
1.00
0.99
1.14
1.41
1.14
1.34
1.66
1.32
2.16
2.01
control
ENS 0.1
ENS 0.5
ENS 1
ENP-S 0.1
ENP-S 0.5
ENP-S 1
ENP-O 0.1
ENP-O 0.5
ENP-O 1
conc. (uM)

[FIG. 8]

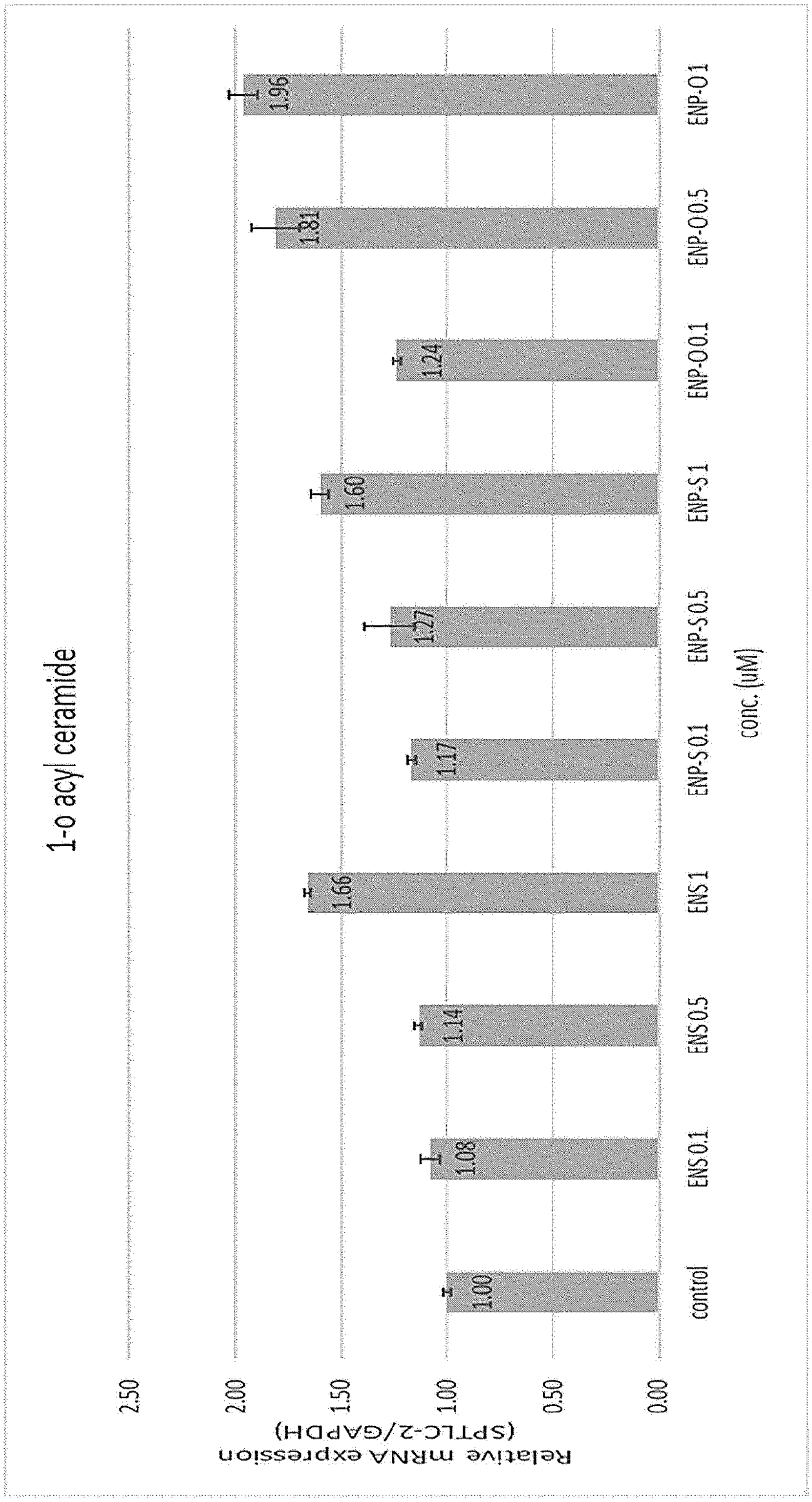
1-o acyl ceramide
Relative mRNA expression (SPTLC-2/GAPDH)
2.50
2.00
1.50
1.00
0.50
0.00
control
ENS 0.1
ENS 0.5
ENS 1
ENP-S 0.1
ENP-S 0.5
ENP-S 1
ENP-O 0.1
ENP-O 0.5
ENP-O 1
1.00
1.08
1.14
1.66
1.17
1.27
1.60
1.24
1.81
1.96
conc. (uM)

[FIG. 9]

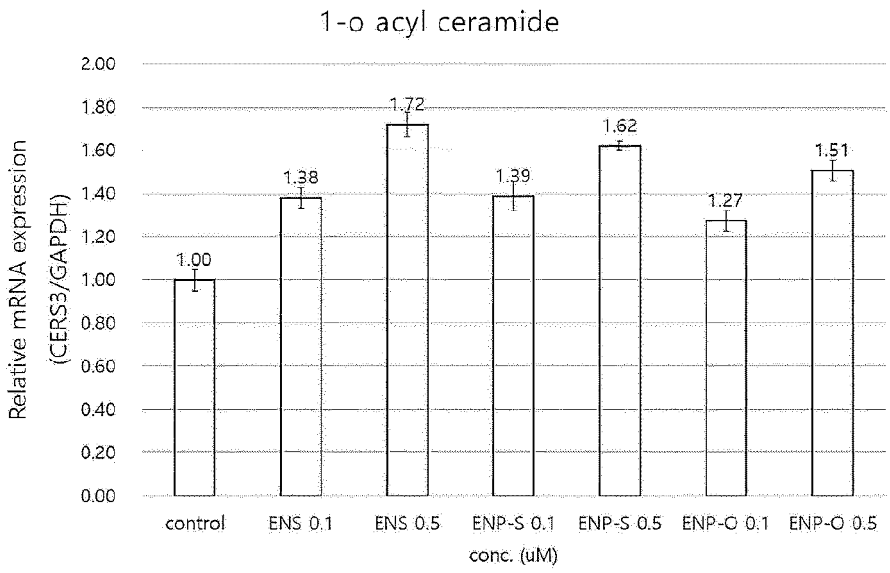
1-o acyl ceramide
Relative mRNA expression (CERS3/GAPDH)
2.00
1.80
1.60
1.40
1.20
1.00
0.80
0.60
0.40
0.20
0.00
1.00
1.38
1.72
1.39
1.62
1.27
1.51
control
ENS 0.1
ENS 0.5
ENP-S 0.1
ENP-S 0.5
ENP-O 0.1
ENP-O 0.5
conc. (uM)

| INTERNATIONAL SEARCH REPORT | International application No. PCT/KR2022/001289 |
|---|---|

**A. CLASSIFICATION OF SUBJECT MATTER**

**C07C 235/76**(2006.01)i; **C07C 231/12**(2006.01)i; **A61K 8/68**(2006.01)i; **A23L 33/10**(2016.01)i; **A61K 31/164**(2006.01)i; **A61P 17/00**(2006.01)i; **A61Q 19/00**(2006.01)i; **A61K 39/00**(2006.01)i

According to International Patent Classification (IPC) or to both national classification and IPC

**B. FIELDS SEARCHED**

Minimum documentation searched (classification system followed by classification symbols)

C07C 235/76(2006.01); A61K 31/133(2006.01); A61K 47/48(2006.01); A61K 9/127(2006.01); A61P 25/00(2006.01); C07C 233/18(2006.01); C07C 233/20(2006.01); C07F 7/18(2006.01); C12P 13/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 세라마이드 (ceramides), 스핑고신 (sphingosine), 피부 (skin), 화장료 조성물 (cosmetic composition)

**C. DOCUMENTS CONSIDERED TO BE RELEVANT**

| Category* | Citation of document, with indication, where appropriate, of the relevant passages | Relevant to claim No. |
|---|---|---|
| X | WO 94-26919 A1 (GIST-BROCADES N.V.) 24 November 1994 (1994-11-24) See claim 9; pages 1, 2, 7 and 8; and example 1. | 1-18 |
| X | KR 10-2011-0019368 A (EVONIK GOLDSCHMIDT GMBH) 25 February 2011 (2011-02-25) See abstract; claims 1-25; paragraphs [0010], [0011], [0083], [0086], [0094], [0097], [0098] and [0117]; and examples 6 and 14. | 1-18 |
| A | KR 10-2004-0051958 A (DOOSAN CORPORATION) 19 June 2004 (2004-06-19) See entire document. | 1-18 |
| A | WO 95-21175 A1 (THE LIPOSOME COMPANY, INC.) 10 August 1995 (1995-08-10) See entire document. | 1-18 |
| A | US 2008-0317767 A1 (BRAXMEIER, T. et al.) 25 December 2008 (2008-12-25) See entire document. | 1-18 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 May 2022** | **09 May 2022** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
**Information on patent family members**

International application No.
**PCT/KR2022/001289**

| Patent document cited in search report | Publication date (day/month/year) | Patent family member(s) | Publication date (day/month/year) |
|---|---|---|---|
| WO 94-26919 A1 | 24 November 1994 | AT 196509 T | 15 October 2000 |
| | | AU 6843494 A | 12 December 1994 |
| | | CA 2139002 A1 | 24 November 1994 |
| | | DE 69425940 T2 | 18 January 2001 |
| | | EP 0651817 A1 | 10 May 1995 |
| | | EP 0651817 B1 | 20 September 2000 |
| | | ES 2152314 T3 | 01 February 2001 |
| | | JP 07-508889 A | 05 October 1995 |
| | | US 5610040 A | 11 March 1997 |
| | | WO 94-26919 A1 | 24 November 1994 |
| KR 10-2011-0019368 A | 25 February 2011 | CN 102057049 A | 11 May 2011 |
| | | CN 102057049 B | 01 October 2014 |
| | | DE 102008002409 A1 | 17 December 2009 |
| | | EP 2283143 A2 | 16 February 2011 |
| | | EP 2283143 B1 | 27 December 2017 |
| | | JP 2011-522550 A | 04 August 2011 |
| | | JP 5647109 B2 | 24 December 2014 |
| | | US 2011-0077302 A1 | 31 March 2011 |
| | | US 8647848 B2 | 11 February 2014 |
| | | WO 2009-150022 A2 | 17 December 2009 |
| | | WO 2009-150022 A3 | 29 April 2010 |
| KR 10-2004-0051958 A | 19 June 2004 | AU 2003-303074 A1 | 09 July 2004 |
| | | WO 2004-054963 A1 | 01 July 2004 |
| WO 95-21175 A1 | 10 August 1995 | AT 195944 T | 15 September 2000 |
| | | AU 1871295 A | 21 August 1995 |
| | | AU 691886 B2 | 28 May 1998 |
| | | CA 2182485 A1 | 10 August 1995 |
| | | DE 69518627 T2 | 11 January 2001 |
| | | DK 0742789 T3 | 13 November 2000 |
| | | EP 0742789 A1 | 20 November 1996 |
| | | EP 0742789 B1 | 30 August 2000 |
| | | EP 1008342 A2 | 14 June 2000 |
| | | EP 1008342 A3 | 29 December 2004 |
| | | ES 2149350 T3 | 01 November 2000 |
| | | FI 116620 B | 13 January 2006 |
| | | GR 3034521 T3 | 29 December 2000 |
| | | JP 09-508900 A | 09 September 1997 |
| | | KR 10-1997-0700679 A | 12 February 1997 |
| | | NO 314405 B1 | 17 March 2003 |
| | | NO 963224 L | 27 September 1996 |
| | | PT 742789 E | 29 December 2000 |
| | | US 5631394 A | 20 May 1997 |
| | | US 5677337 A | 14 October 1997 |
| | | US 5681589 A | 28 October 1997 |
| US 2008-0317767 A1 | 25 December 2008 | AT 442865 T | 15 October 2009 |
| | | AU 2005-231622 A1 | 20 October 2005 |
| | | AU 2005-231622 B2 | 26 May 2011 |
| | | CA 2562266 A1 | 20 October 2005 |
| | | EP 1732612 A1 | 20 December 2006 |
| | | EP 1732612 B1 | 16 September 2009 |

Form PCT/ISA/210 (patent family annex) (July 2019)

**INTERNATIONAL SEARCH REPORT**
**Information on patent family members**

International application No.
**PCT/KR2022/001289**

| Patent document cited in search report | Publication date (day/month/year) | Patent family member(s) | | | Publication date (day/month/year) |
|---|---|---|---|---|---|
| | | EP | 2119455 | A1 | 18 November 2009 |
| | | JP | 2007-532512 | A | 15 November 2007 |
| | | JP | 4896870 | B2 | 14 March 2012 |
| | | WO | 2005-097199 | A1 | 20 October 2005 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020210021447
- KR 101816019
- KR 1020170084950


### Non-patent literature cited in the description


- *International Journal of Cosmetic Science,* 2017, vol. 39, 366-372